# EUROPEAN PATENT SPECIFICATION

(11) **EP 2 433 561 B1**
(45) Date of publication and mention of the grant of the patent: **12.11.2014**
(21) Application number: 10180444.1
(22) Date of filing: 27.09.2010
(51) Int. Cl.: A61B 5/0492, A61B 5/0408, A61N 1/04

(54) **Garment with three-dimensional wearable electrode set**
Kleidungsstück mit dreidimensionalem tragbarem Elektrodensatz
Vêtement avec jeu d'électrodes tridimensionnelles portables

(43) Date of publication of application: 28.03.2012
(73) Proprietor: Tex-Ray Industrial Co., Ltd., Taipei City (TW); King's Metal Fiber Technologies Co., Ltd., Taipei City (TW)
(72) Inventor: Lee, James, Taipei City (TW); Huang, Hong-Hsu, Taipei City (TW)
(74) Representative: Michalski Hüttermann & Partner Patentanwälte mbB

(56) References cited:
- EP-A1- 2 177 156
- EP-A1- 2 191 737
- WO-A1-2006/095279
- WO-A1-2009/020274
- WO-A2-2008/032282
- WO-A2-2009/013704
- US-A- 4 729 377
- US-A- 5 309 916

## Description

### BACKGROUND OF THE INVENTION

### Field of the Invention

The present invention provides a three-dimensional (3D) wearable electrode set. More specifically, the present invention provides a three-dimensional wearable electrode set without electric adhesive patches.

### Descriptions of the Related Art

In medical domain, it is important to keep tracking patients' physiological statuses, such as myoclectricity status or cardiac reflex status. Some portable electronic devices for sensing people's physiological statuses have been proposed in the prior patent applications, such as US 4,729,377 A, which discloses the preamble of claim 1, EP 2177156 A1, US 5,309,916 A, WO 2006/095279 A1, EP 2191737 A1, WO 2009/13704 and WO 2009/20274, so that the electrical monitoring of sites or electrical stimulation applied to human body can be performed. However, those conventional portable electronic devices arc inconvenient and not suitable for users (e.g. sportsman) when the users take exercise in some situations.

In general, the myoelectricity status is sensed according to electromyography (EMG) signals, and the cardiac reflex status is sensed according to electrocardiograph (ECG) signals.

Typically, the EMG signals or ECG signals are able to be measured via a plurality of electric adhesive patches adhered on a human body. More specifically, these electric adhesive patches are adhered on different portions of the human body to sense the EMG signals or ECG signals, and are electrically connected to a monitor to analyze the signals for displaying.

Furthermore, to be more convenient, the industry integrates these electric adhesive patches with a garment, such as a T-shirt. In other words, these electric adhesive patches are adhered on the garment and in close contact with the skin of the human body, so that for sportsmen, they can be notified whether the exercises is effective or not by checking the ECG signals while they are taking exercises.

However, the electric adhesive patches will lose their adhesive and cause folder over in such a manner that the physiological signals can not be measured or are not reliable. In fact, there is a high possibility for sportsmen that the electric adhesive patches fall off from the garment during their exercises.

In view of this, it is important to provide wearable electrodes that are convenient for use and are not requiring the electric adhesive patches.

### SUMMARY OF THE INVENTION

The invention is defined in the appended claims.

Accordingly, the 3D wearable electrode set comprising the first and second ring electrodes do not require electric adhesive patches to sense the myoelectricity status or the cardiac reflex status of a human body, and are able to cover around different portions of the human body which may give a better and more accurate results than the electric adhesive patches of the prior art.

The detailed technology and preferred embodiments implemented for the subject invention are described in the following paragraphs accompanying the appended drawings for people skilled in this field to well appreciate the features of the claimed invention.

### BRIEF DESCRIPTION OF THE DRAWINGS

**FIG 1** is a schematic view illustrating a preferred embodiment;
**FIGs. 2A-2C** are schematic views illustrating the first and second ring electrodes of the preferred embodiment;
**FIG 3A** is a schematic view illustrating conductive fabric of the preferred embodiment;
**FIG. 3B** is a schematic view illustrating insulating fabric of the preferred embodiment; and
**FIGs. 4A-4B** are schematic views illustrating the first and second ring electrodes of another preferred embodiment.

### DESCRIPTION OF THE PREFERRED EMBODIMENT

In the following description, this invention will be explained with reference to embodiments. However, these embodiments are not intended to limit this invention to any specific environment, applications or implementations described in these embodiments. Therefore, description of these embodiments is only for purposes of illustration rather than to limit the present invention. It should be appreciated that in the following embodiments and the attached drawings, the elements not related directly to **this** invention are omitted from depiction.

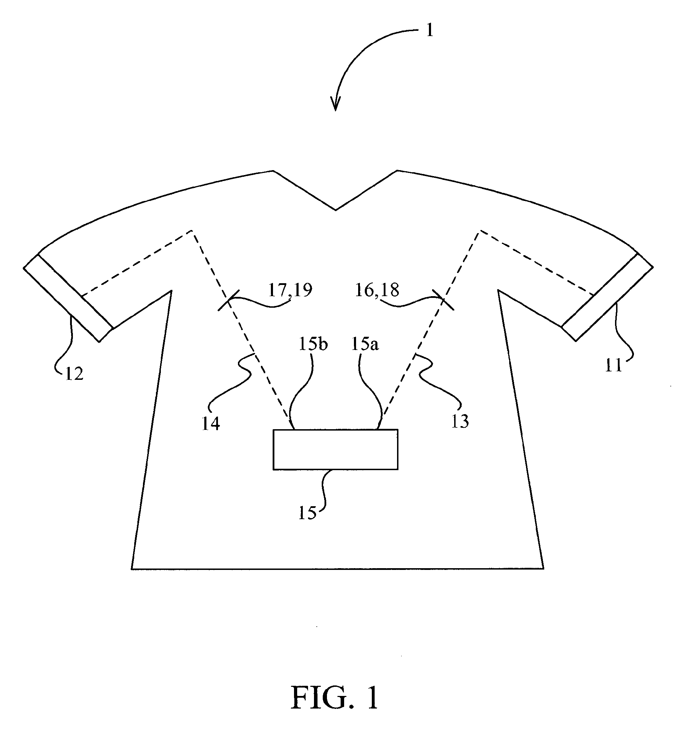

**FIG 1** is a schematic view illustrating a preferred embodiment of a garment **1** incorporated with a 3D wearable electrode set of the present invention. The 3D wearable electrode set comprises a first ring electrode **11** and a second ring electrode **12**. The first ring electrode **11** and the second ring electrode **12** may be sewed on the two sleeves of the garment **1.** In other embodiments, the first ring electrode **11** and the second ring electrode **12** may form with the garment **1** as a piece rather than an addition of the garment **1.** The first ring electrode **11** is electrically connected to a first terminal **15a** of a processor **15** via a first conductive thread **13,** and the second ring electrode **12** is electrically connected to a second terminal **15b** of the processor **15** via a second conductive thread **14.** In this preferred embodiment, the first conductive thread **13** and the second conductive tread **14** are yarns blendcd with the conductive fibers and the insulating fibers. It should be noted that the first conductive thread **13** and the second conductive thread **14** are made by pure conductive fibers, conductive wires, or conductive inks, the present description has no intention to limit the material of the first conductive thread **13** and the second conductive thread **14** as long as their material are conductive.

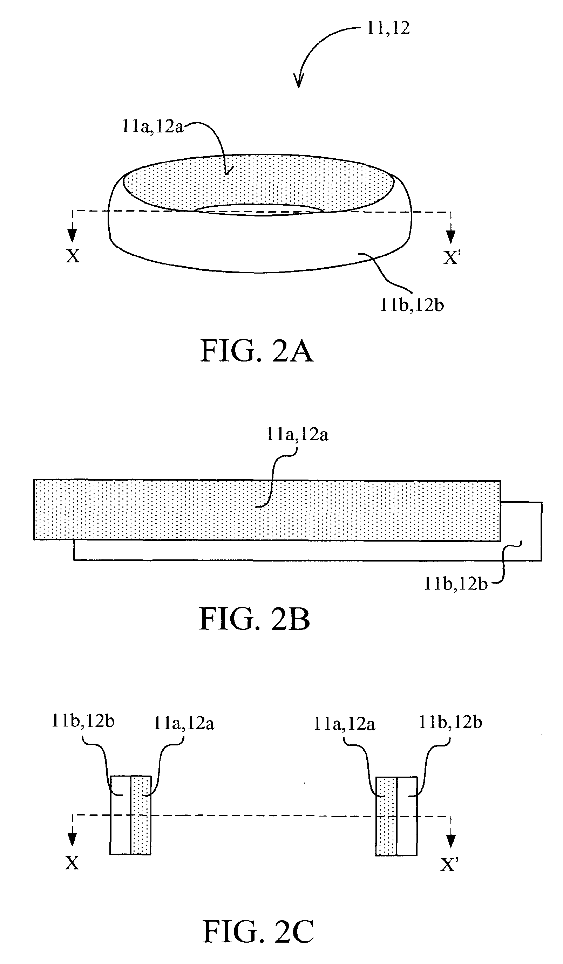

**FIGs. 2A-2C** are schematic views of the first ring electrode **11** or the second ring electrode **12** in accordance with the garment **1** of the preferred embodiment. More specifically, **FIG. 2A** is a schematic views enlarging the first ring electrode **11** or the second ring electrode **12,** **FIG 2B** is an exploded view of different layers of the first ring electrode **11** or the second ring electrode **12,** and **FIG. 2C** is a cross sectional view of the first ring electrode **11** or the second ring electrode **12** in accordance with **X-X'**.

The first ring electrode **11** has a first conductive layer **11a** and a first ring basis **11b,** wherein the first conductive layer **ll a** is attached to the first ring basis **11b,** and the first conductive layer **11a** is woven with conductive fibers and insulating fibers while the first ring basis **11b** is formed with insulating fabric.

Similarly, the second ring electrode **12** has the same structure as the first ring electrode **11.** In other words, the second ring electrode **12** has a second conductive layer **12a** and a second ring basis **12b,** wherein the second conductive layer **12a** is attached to the second ring basis **12b,** and the second conductive layer **12a** is woven with conductive fibers and insulating fibers while the second ring basis **12b** is formed with insulating fabric.

In this preferred embodiment, the first conductive layer **11a** has a ring structure, and a surface area of that is equal to a surface area of the first ring basis **11b.** The second conductive layer **12a** also has the ring structure, and a surface area of that is equal to a surface area of the second ring basis **12b.**

In **FIG 2C****,** the first conductive layer **11a** and the first ring basis **11b** on the right side of **FIG. 2C** are refer to a right portion of the first ring electrode **11** in **FIG. 2A****.** Similarly, the second conductive layer **12a** and the second ring basis **12b** on the right side of **FIG. 2C** are refer to a right portion of the second ring electrode **12** in **FIG. 2A**.

On the other hand, the first conductive layer **11a** and the first ring basis **11b** on the left side of **FIG. 2C** are refer to a left portion of the second ring electrode **12** in **FIG. 2A****,** and the second conductive layer **12a** and the second ring basis **12b** on the left side of **FIG. 2C** are refer to a left portion of the second ring electrode **12** in **FIG. 2A****.**

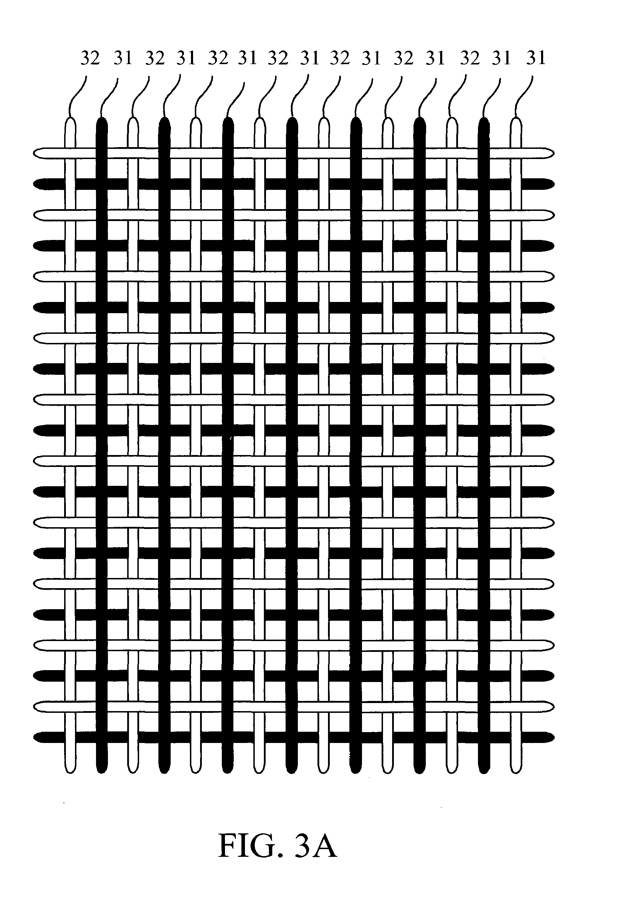

Furthermore, the first conductive layer **11a** and the second conductive layer **12a** are woven with conductive fibers **31** and insulating fibers **32** in such a manner as shown in **FIG. 3A****,** wherein material of the conductive fibers **31** is metal fibers with electric conductively, such as stainless steel. In other embodiments, the first conductive layer **11a** and the second conductive layer **12a** may be formed with fabric applied conductive ink or conductive paint.

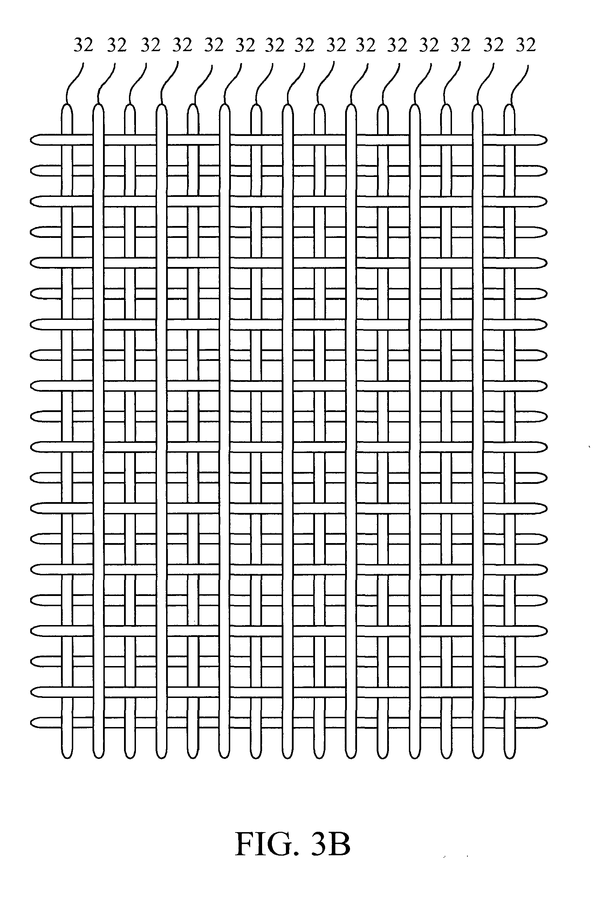

On the other hand, the first ring basis **11b** and the second ring basis **12b** are woven with a plurality of insulating fibers **32** in such a manner as shown in **FIG 3B****,** wherein material of the insulating fibers **31** is conventional fibers without electric conductively, such as cotton fibers. It should be noted that the first conductive layer **11a** and the second conductive layer **12a** can be made by conductive inks, the present description has no intention to limit the material of the first conductive layer **11a** and the second conductive layer **12a** as long as their material are conductive.

Please refer to **FIGs. 1****~2C,** the 3D wearable electrode set is for a human body. The first ring electrode **11** and the second ring electrode **12** are made by elastic material, so that the first ring electrode **11** and the second ring electrode **12** are able to slight tight round a first portion (e.g. the right elbow) and a second portion (e.g. a left elbow) of the human body respectively.

Further speaking, the first conductive layer **11a** and the second conductive layer **12a** contact with the skin of the first potion and the second portion of the human body and are electrically connected to the first terminal **15a** and the second terminal **15b** of the processor **15** via the first conductive thread **13** and the second conductive thread **14** respectively. Therefore, the human body may receive some electric stimulation from the processor **15** via the first conductive layer **11a** and the second conductive layer **12a.**

For example, the 3D wearable electrode set may be for diathermy, such as transcutaneous electrical nerve stimulation (TENS). The processor **15** may generate a first simulation current **16** and a second simulation current **17.** The first simulation current **16** is transmitted to the first conductive layer **11a** of the first ring electrode **11** via the first conductive thread **13,** and the second simulation current **17** is transmitted to the second conductive layer **12a** of the second ring electrode **12** via the second conductive thread **14.** According to the first simulation current **16** and the second simulation current **17** from the processor **15,** the diathermy can be easily achieved by the processor **15**, the first ring electrode **11** and the second ring electrode **12.**

On the other hand, if the 3D wearable electrode set is for monitoring the myoclectricity status or the cardiac reflex status of the human body, the first conductive layer **11a** may receive a first electrical impulse **18** (e.g. one of the EMG signals or one of the ECG signals) generated from the first portion of the human body, and then the first electrical impulse **18** is transmitted to the first terminal **15a** of the processor **15** via the first conductive thread **13.** Similarly, the second conductive layer **12a** may receive a second electrical impulse **19** (e.g. another EMG signal or another ECG signal) generated from the second portion of the human body, and then the second electrical impulse **19** is transmitted to the second terminal **15b** of the processor **15** via the second conductive thread **14.**

After receiving the first electrical impulse **18** and the second electrical impulse **19,** the processor **15** analyzes the impulses **18, 19** to retrieve the electromyogram or the electrocardiogram of the human body. According to the first electrical impulse **18** and the second electrical impulse **19** from the first ring electrode **11** and the second ring electrode **12**, monitoring of the myoelectricity status or the cardiac reflex status of the human body can be easily achieved by the processor **15**, the first ring electrode **11** and the second ring electrode **12**. In other embodiments, the first ring electrode **11** and the second ring electrode **12** may additionally sense heart beat pulse.

It should be noted that even though the 3D wearable electrode set are incorporated with garment **1** in this preferred embodiment as shown in **FIG. 1**, the 3D wearable electrode set may incorporated with a long sleeve sweater, or a sport pant for allow to cover around arms, feet, thigh, belly or even neck of the body. Furthermore, to obtain a better and more accurate the myoelectricity status or the cardiac reflex status, the 3D wearable electrode set must comprises at lest two ring electrodes to cover around different portions of the human body, people skilled in this art may rapidly add more ring electrodes since the structures of ring electrodes are basically the same. For the cardiac reflex status, it is even desired to place the ring electrodes across the heart, for example, the ring electrodes are placed at right elbow and left ankle.

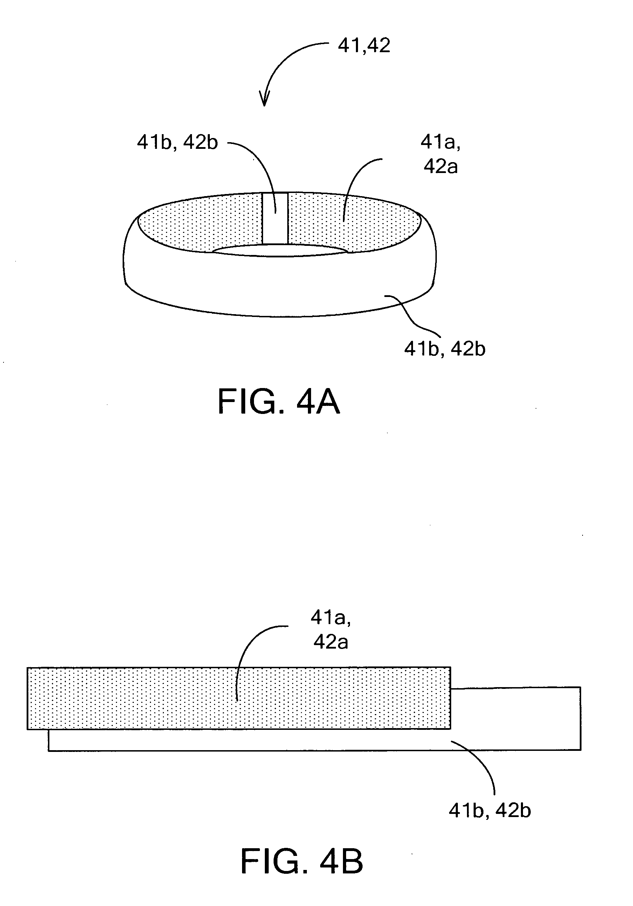

**FIGs. 4A-4B** are schematic views of an first ring electrode **41** or a second ring electrode **42** of another preferred embodiment. More specifically, **FIG. 4A** is a schematic view enlarging the first ring electrode **41** or the second ring electrode **42,** and **FIG 4B** is an exploded view of different layers of the first ring electrode **41** or the second ring electrode **42.** The first ring electrode **41** or the second ring electrode **42** may combined with the garment **1** of the prior preferred embodiment, the details are described as above and therefore will not be mentioned here.

The first ring electrode **41** has a first conductive layer **41a** and a first ring basis **41b,** wherein a surface area of the first conductive layer **41a** is less than a surface area of the first ring basis **41b,** and the first conductive layer **41a** is attached to a part of the first ring basis **41b.** Preferably, the surface area of the first conductive layer **41a** holds a ratio between 20% and 80% of the surface area of the first ring basis **41b.** The first conductive layer **41a** is woven with conductive fibers and insulating fibers while the first ring basis **41b** is formed with insulating fabric.

Similarly, the second ring electrode **42** has the same structure as the first ring electrode **41.** In other words, the second ring electrode **42** has a second conductive layer **42a** and a second ring basis **42b.** A surface area of the second conductive layer **42a** is less than a surface area of the second ring basis **42b,** and the second conductive layer **42a** is attached to a part of the second ring basis **42b.** Preferably, a surface area of the second conductive layer **42a** holds a ratio between 20% and 80% of a surface area of the second ring basis **42b.** The second conductive layer **42a** is woven with conductive fibers and insulating fibers while the second ring basis **42b** is formed with insulating fabric.

Because the surface area of the first conductive layer **41a**/the second conductive layer **42a** is less than the surface area of the first ring basis **41b**/the second ring basis **42b,** so that a conductive area of the first ring electrode **41** or the second ring electrode **42** are discontinuous as shown in **FIG. 4A**, which is different from first ring electrode **11** and the second ring electrode **12** as shown in **FIG. 2A**.

The first conductive layer **41a** and the second conductive layer **42a** are woven with conductive fibers **31** and insulating fibers **32** in such a manner as shown in **FIG. 3A****,** wherein material of the conductive fibers **31** is metal fibers with electric conductively, such as stainless steel. The first ring basis **41b** and the second ring basis **42b** are woven with a plurality of insulating fibers **32** in such a manner as shown in **FIG 3B****,** wherein material of the insulating fibers 31 is conventional fibers without electric conductively, such as cotton fibers.

Similarly, the first conductive layer **41a** and the second conductive layer **42a** may contact with the skin of the first potion and the second portion of the human body and are electrically connected to the first terminal (not shown) and the second terminal (not shown) of the processor (not shown) via the first conductive thread (not shown) and the second conductive thread (not shown) respectively. Therefore, the human body may receive some electric stimulation from the processor via the first conductive layers and the second conductive layers. The details for TENS and monitoring the myoelectricity status or the cardiac reflex status of the human body applications are already described in the previous preferred embodiment and therefore will not be mentioned here.

Accordingly, the 3D wearable electrode set which comprises the first ring electrode and the first ring electrode does not require electric adhesive patches, and is able to cover around different portions of the human body which may give a better and more accurate results than the electric adhesive patches of the prior art. Hence, the problem of the prior art is overcome.

The above disclosure is related to the detailed technical contents and inventive features thereof. People skilled in this field may proceed with a variety of modifications and replacements based on the disclosures and suggestions of the invention as claimed. Nevertheless, although such modifications and replacements arc not fully disclosed in the above descriptions, the claimed invention is not limited to the above disclosure.

## Claims

1. A garment with two sleeves, the garment incorporated with a three-dimensional (3D) wearable electrode set for a human body without electric adhesive patches, wherein the electrode set comprises
- a processor (15);
- a first ring electrode (11), having a first conductive layer (11 a) and a first ring basis (11 b), wherein the first conductive layer (11 a) is formed with conductive material, the first ring basis (11 b) is formed with insulating fabric, and the first conductive layer (11 a) is attached to the first ring basis (11 b); and
- a second ring electrode (12), having a second conductive layer (12a) and a second ring basis (12a), wherein the second conductive layer (12a) is formed with the conductive material, the second ring basis (12a) is formed with the insulating fabric, and the second conductive layer (12a) is attached to the second ring basis (12a),
wherein
the first conductive layer (11 a) is electrically connected to a first terminal (15a) of the processor via a first conductive thread (13), and the second conductive layer (12a) is electrically connected to a second terminal (15b) of the processor (15) via a second conductive thread (14);
the processor (15) is configured to generate a first stimulation current (16) and a second stimulation current (17), the first stimulation current (16) being transmitted to the first conductive layer (11a) of the first ring electrode (11) via the first conductive thread (13), the second stimulation current (17) being transmitted to the second conductive layer (12a) of the second ring electrode (12) via the second conductive thread (14), wherein diathermy is achieved by the processor (15), the first ring electrode (11) and the second ring electrode (12) according to the first stimulation current (16) and the second stimulation current (17);
the first conductive layer (11 a) is further configured to receive a first electrical impulse (18) generated from the right elbow of the human body, and to transmit the first electrical impulse (18) to the first terminal (15a) of the processor (15) via the first conductive thread (13),
the second conductive layer (12a) is further configured to receive a second electrical impulse (19) generated from the left elbow of the human body, and
to transmit the second electrical impulse (19) to the second terminal (15b) of the processor (15) via the second conductive thread (14), and
the processor (15) is further configured to analyse the impulses (18, 19) to retrieve the electromyogram or the electrocardiogram of the human body and to monitor the myoelectricity status or the cardiac reflex status of the human body according to the first electrical impulse (18) and the second electrical impulse (19) from the first ring electrode (11) and the second ring electrode (12);
**characterised in that**
the first ring electrode and the second ring electrode are sewed on the two sleeves of the garment, respectively;
the first ring electrode (11) is adapted for covering around the right elbow of the human body, and the second ring electrode (12) is adapted for covering around the left elbow of the human body; and
the first ring electrode (11) and the second ring electrode (12) are made by elastic material so that they are able to slight tight round the right elbow and the left elbow of the human body, respectively.

2. The garment of claim 1, wherein the conductive material is a fabric woven with conductive fibers and insulating fibers.

3. The garment of any of claims 1 to 2, wherein a surface area of the first conductive layer (41a) is less than a surface area of the first ring basis (41b), and a surface area of the second conductive layer (42a) is less than a surface area of the second ring basis (42b).

4. The garment of any of claims 1 to 2, wherein the first conductive layer (11a) substantially has a ring structure, a surface area of the first conductive layer (11a) is equal to a surface area of the first ring basis (11b), the second conductive layer (12a) substantially has the ring structure, and a surface area of the second conductive layer (12a) is equal to a surface area of the second ring basis (12b).

## Patentansprüche

1. Kleidungsstück mit zwei Ärmeln, wobei das Kleidungsstück einen tragbaren dreidimensionalen (3D) Elektrodensatz für einen menschlichen Körper ohne elektrische Klebstoffabschnitte aufweist, wobei der Elektrodensatz aufweist:
- einen Prozessor (15);
- eine erste Ringelektrode (11) mit einer ersten leitfähigen Lage (11a) und einer ersten Ringbasis (11b), wobei die erste leitfähige Lage (11a) aus einem leitfähigen Material ausgebildet ist, die erste Ringbasis (11b) aus einem isolierenden Textilmaterial ausgebildet ist und die erste leitfähige Lage (11a) an der ersten Ringbasis (11 b) befestigt ist; und
- eine zweite Ringelektrode (12) mit einer zweiten leitfähigen Lage (12a) und einer zweiten Ringbasis (12b), wobei die zweite leitfähige Lage (12a) aus dem leitfähigen Material ausgebildet ist, die zweite Ringbasis (12b) aus dem isolierenden Textilmaterial ausgebildet ist und die zweite leitfähige Lage (12a) an der zweiten Ringbasis (12b) befestigt ist, wobei
die erste leitfähige Lage (11a) über einen ersten leitfähigen Faden (13) mit einem ersten Anschluss (15a) des Prozessors verbunden ist und die zweite leitfähige Lage (12a) über einen zweiten leitfähigen Faden (14) mit einem zweiten Anschluss (15b) des Prozessors (15) verbunden ist,
der Prozessor (15) dafür konfiguriert ist, einen ersten Reizstrom (16) und einen zweiten Reizstrom (17) zu erzeugen, wobei der erste Reizstrom (16) über den ersten leitfähigen Faden (13) zur ersten leitfähigen Lage (11a) der ersten Ringelektrode (11) übertragen wird und der zweite Reizstrom (17) über den zweiten leitfähigen Faden (12a) zur zweiten leitfähigen Lage (12a) der zweiten Ringelektrode (12) übertragen wird, so dass durch den Prozessor (15), die erste Ringelektrode (11) und die zweite Ringelektrode (12) gemäß dem ersten Reizstrom (16) und dem zweiten Reizstrom (17) eine Diathermie erzielt wird,
die erste leitfähige Lage (11a) ferner dafür konfiguriert ist, einen vom rechten Ellbogen des menschlichen Körpers erzeugten ersten elektrischen Impuls zu empfangen und den ersten elektrischen Impuls (18) über den ersten leitfähigen Faden (13) an den ersten Anschluss (15a) des Prozessors (15) zu übertragen,
die zweite leitfähige Lage (12a) ferner dafür konfiguriert ist, einen vom linken Ellbogen des menschlichen Körpers erzeugten zweiten elektrischen Impuls (19) zu empfangen und den zweiten elektrischen Impuls (19) über den zweiten leitfähigen Faden (14) an den zweiten Anschluss (15b) des Prozessors (15) zu übertragen, und
der Prozessor (15) ferner dafür konfiguriert ist, die Impulse (18, 19) zu analysieren, um das Elektromyogramm oder das Elektrokardiogramm des menschlichen Körpers zu erfassen und den myoelektrischen Status oder den kardialen Reflexstatus des menschlichen Körpers gemäß dem ersten elektrischen Impuls (18) und dem zweiten elektrischen Impuls (19) von der ersten Ringelektrode (11) und der zweiten Ringelektrode (12) zu überwachen;
**dadurch gekennzeichnet, dass**
die erste Ringelektrode und die zweite Ringelektrode auf die beiden Ärmel des Kleidungsstücks aufgenäht sind;
die erste Ringelektrode (11) dazu geeignet ist, den rechten Ellbogen des menschlichen Körpers zu umschließen, und die zweite Ringelektrode (12) dazu geeignet ist, den linken Ellbogen des menschlichen Körpers zu umschließen; und
die erste Ringelektrode (11) und die zweite Ringelektrode (12) aus einem elastischen Material ausgebildet sind, so das sie dazu geeignet sind, den rechten Ellbogen bzw. den linken Ellbogen des menschlichen Körpers leicht gespannt zu umschließen.

2. Kleidungsstück nach Anspruch 1, wobei das leitfähige Material ein Webstoff mit leitfähigen Fasern und isolierenden Fasern ist.

3. Kleidungsstück nach Anspruch 1 oder 2, wobei eine Oberfläche der ersten leitfähigen Lage (41a) kleiner ist als eine Oberfläche der ersten Ringbasis (41 b), und wobei eine Oberfläche der zweiten leitfähigen Lage (42a) kleiner ist als eine Oberfläche der zweiten Ringbasis (42b).

4. Kleidungsstück nach Anspruch 1 oder 2, wobei die erste leitfähige Lage (11a) im Wesentlichen eine Ringstruktur hat, wobei eine Oberfläche der ersten leitfähigen Lage (11a) einer Oberfläche der ersten Ringbasis (11 b) gleicht, und wobei die zweite leitfähige Lage (12a) im Wesentlichen eine Ringstruktur hat, wobei die Oberfläche der zweiten leitfähigen Lage (12a) der Oberfläche der zweiten Ringbasis (12b) gleicht.

## Revendications

1. Vêtement avec deux manches, le vêtement étant incorporé dans un ensemble d'électrodes portables tridimensionnel (3D) pour un corps humain, sans patch adhésif électrique, dans lequel l'ensemble d'électrodes comprend
- un processeur (15) ;
- une première électrode annulaire (11) possédant une première couche conductrice (11a) et une première base annulaire (11b), la première couche conductrice (11a) étant formée avec un matériau conducteur, la première base annulaire (11b) étant formée avec un tissu isolant, et la première couche conductrice (11a) étant reliée à la première base annulaire (11b); et
- une deuxième électrode annulaire (12) possédant une deuxième couche conductrice (12a) et une deuxième base annulaire (12a), la deuxième couche conductrice (12a) étant formée avec le matériau conducteur, la deuxième base annulaire (12a) étant formée avec le tissu isolant, et la deuxième couche conductrice (12a) étant reliée à la deuxième base annulaire (12a),
dans lequel
la première couche conductrice (11a) est électriquement reliée à un premier terminal (15a) du processeur, par le biais d'un premier fil conducteur (13), et la deuxième couche conductrice (12a) est électriquement reliée à un deuxième terminal (15b) du processeur (15) par le biais d'un deuxième fil conducteur (14) ;
le processeur (15) est configuré pour générer un premier courant de stimulation (16) et un deuxième courant de stimulation (17), le premier courant de stimulation (16) étant transmis à la première couche conductrice (11a) de la première électrode annulaire (11) par le biais du premier fil conducteur (13), le deuxième courant de stimulation (17) étant transmis à la deuxième couche conductrice (12a) de la deuxième électrode annulaire (12) par le biais du deuxième fil conducteur (14), une diathermie étant établie le processeur (15), la première électrode annulaire (11) et la deuxième électrode annulaire (12) selon le premier courant de stimulation (16) et le deuxième courant de stimulation (17) ;
la première couche conductrice (11a) est en outre configurée pour recevoir une première impulsion électrique (18) générée depuis le coude droit du corps humain, et pour transmettre la première impulsion électrique (18) au premier terminal (15a) du processeur (15) par le biais du premier fil conducteur (13),
la deuxième couche conductrice (12a) est en outre configurée pour recevoir une deuxième impulsion électrique (19) générée depuis le coude gauche du corps humain, et
pour transmettre la deuxième impulsion électrique (19) au deuxième terminal (15b) du processeur (15) par le biais du deuxième fil conducteur (14), et
le processeur (15) est en outre configuré pour analyser les impulsions (18, 19) pour extraire l'électromyogramme ou l'électrocardiogramme du corps humain et pour surveiller l'état de myoélectricité ou l'état de réflexe cardiaque du corps humain en fonction de la première impulsion électrique (18) et de la deuxième impulsion électrique (19) émises par la première électrode annulaire (11) et la deuxième électrode annulaire (12) ;
**caractérisé en ce que**
la première électrode annulaire et la deuxième électrode annulaire sont cousues respectivement sur les deux manches du vêtement ;
la première électrode annulaire (11) est adaptée pour couvrir le pourtour du coude droit du corps humain, et la deuxième électrode annulaire (12) est adaptée pour couvrir le pourtour du coude gauche du corps humain ; et
la première électrode annulaire (11) et la deuxième électrode annulaire (12) sont constituées d'une matière élastique, de façon à pouvoir s'appliquer étroitement autour du coude droit et du coude gauche du corps humain, respectivement.

2. Vêtement selon la revendication 1, dans lequel le matériau conducteur est un tissu avec des fibres conductrices et des fibres isolantes.

3. Vêtement selon l'une quelconque des revendications 1 à 2, dans lequel une aire de surface de la première couche conductrice (41a) est inférieure à une aire de surface de la première base annulaire (41b), et une aire de surface de la deuxième couche conductrice (42a) est inférieure à une aire de surface de la deuxième base annulaire (42b).

4. Vêtement selon l'une quelconque des revendications 1 à 2, dans lequel la première couche conductrice (11a) possède substantiellement une structure annulaire, une aire de surface de la première couche conductrice (11a) est égale à une aire de surface de la première base annulaire (11b), la deuxième couche conductrice (12a) possède substantiellement une structure annulaire, et une aire de surface de la deuxième couche conductrice (12a) est égale à une aire de surface de la deuxième base annulaire (12b).
